## Europäisches Patentamt

## European Patent Office

## Office européen des brevets

(19)

(11) Veröffentlichungsnummer : **0 062 305**
**B1**

(12) **EUROPÄISCHE PATENTSCHRIFT**

(45) Veröffentlichungstag der Patentschrift :
13.06.84

(21) Anmeldenummer : 82102753.9

(22) Anmeldetag : 01.04.82

(51) Int. Cl.³ : **C 07 C 51/08, C 07 C 59/185**

(54) **Verfahren zur Herstellung von 5-Oxohexansäure.**

(30) Priorität : 04.04.81 DE 3113626

(43) Veröffentlichungstag der Anmeldung :
13.10.82 Patentblatt 82/41

(45) Bekanntmachung des Hinweises auf die Patenterteilung : 13.06.84 Patentblatt 84/24

(84) Benannte Vertragsstaaten :
BE CH DE FR GB IT LI NL

(56) Entgegenhaltungen :
FR-A- 1 280 596
US-A- 3 542 822

(73) Patentinhaber : **HOECHST AKTIENGESELLSCHAFT**
**Postfach 80 03 20**
**D-6230 Frankfurt am Main 80 (DE)**

(72) Erfinder : **Mack, Karl Ernst, Dr.**
**Klingenbachstrasse 43**
**D-6200 Wiesbaden (DE)**
Erfinder : **Müller, Werner Heinrich, Dr.**
**Taunusblick 5**
**D-6239 Eppstein/Taunus (DE)**

EP 0 062 305 B1

## Beschreibung

5-Oxohexansäure ist eine begehrte bifunktionelle Verbindung, die vielseitig Verwendung findet, u. a. zur Herstellung von korrosionshemmenden Substanzen (US-PS 3 382 081), von Antioxidantien (DE-OS 2 544 014), von Isomerisierungsstabilisatoren (US-PS 3 808 338) und von Chinazolinderivaten (US-PS 3 383 524). Schließlich wird 5-Oxohexansäure durch einstufige Umwandlung an Katalysatoren in Resorcin überführt (DE-OS 2 450 086). 5-Oxohexansäurenitril wird großtechnisch durch sogenannte Cyanethylierung von Aceton mit Acrylnitril hergestellt (DE-OS 2 329 923).

Die Umwandlung von 5-Oxohexansäurenitril in 5-Oxohexansäure mittels wäßrigem Alkalihydroxid ist bekannt (JACS 74 (1952) Seite 5597), führt jedoch zunächst zum Alkalisalz der 5-Oxohexansäure, aus dem in einem zweiten Verfahrensschritt die freie Säure durch Zusatz einer starken Mineralsäure freigesetzt werden muß, wobei jedoch äquimolare Mengen des Alkalisalzes der Mineralsäure entstehen. Es war daher wünschenswert, ein Verfahren zu entwickeln, das die Umwandlung von 5-Oxohexansäurenitril in 5-Oxohexansäure einstufig durch direkte Einwirkung einer wäßrigen, starken Mineralsäure unter gleichzeitiger Bildung des Ammoniumsalzes der Mineralsäure als Coprodukt ermöglicht. Derartige Ammoniumsalze können zu wertvollen, Stickstoff enthaltenden Düngemitteln aufgearbeitet werden.

Gemäß DE-AS 2 144 170 werden 5-Oxocarbonsäurenitrile durch Einwirkung von wäßriger Mineralsäure in hoher Ausbeute zu Cyclohexandionen umgesetzt.

Überraschenderweise wurde nun gefunden, daß das Nitril der 5-Oxohexansäure in Gegenwart von wäßriger Mineralsäure in hoher Ausbeute in 5-Oxohexansäure umgewandelt wird und nicht in Cyclohexandion.

Gegenstand der Erfindung ist daher ein Verfahren zur Herstellung von 5-Oxohexansäure, das dadurch gekennzeichnet ist, daß 5-Oxohexansäurenitril mit 2 bis 30 Mol Wasser und 0,1 bis 5 Mol einer starken Mineralsäure je Mol 5-Oxohexansäurenitril bei Temperaturen von 20 bis 200 °C umgesetzt wird.

Vorzugsweise verwendet man 2 bis 15 Mol Wasser und 0,5 bis 2 Mol Säure je Mol Nitril, insbesondere 2 bis 5 Mol Wasser und 0,75 bis 1,5 Mol Säure je Mol Nitril.

Die Reaktionstemperatur beträgt vorzugsweise 50 bis 150 °C, insbesondere 80 bis 130 °C.

Als starke Mineralsäuren sind z. B. Salzsäure, Schwefelsäure und Phosphorsäure geeignet, besonders Schwefelsäure.

Die Reihenfolge des Zusammenbringens der Reaktionspartner ist nicht kritisch. Vorzugsweise jedoch wird die wäßrige Mineralsäure portionsweise zum 5-Oxohexansäurenitril hinzugefügt.

Nach erfolgter Reaktion kann die Aufarbeitung nach herkömmlichen Methoden durchgeführt werden, beispielsweise dadurch, daß die Reaktionslösung mit einem mit Wasser nicht mischbaren organischen Extraktionsmittel wie Chlorbenzol extrahiert wird und nach Trennen in wäßrige und organische Phase aus der letzteren das Extraktionsmittel destillativ zurückgewonnen und danach 5-Oxohexansäure von unumgesetztem 5-Oxohexansäurenitril destillativ abgetrennt wird. Die wäßrige Phase kann entweder verworfen oder nach bekannten Methoden aufgearbeitet werden.

· Außer Chlorbenzol eignen sich als Extraktionsmittel auch andere halogenierte Kohlenwasserstoffe wie Methylenchlorid, Chloroform, Tetrachlorkohlenstoff, Trichlorethan, Tetrachlorethan oder Ether wie Diisopropylether oder Ketone wie Methylisobutylketon und Cyclohexanon, sowie Aromaten wie Benzol, Toluol, Xylole oder Nitrobenzol.

Durch Anwendung des erfindungsgemäßen Verfahrens wird 5-Oxohexansäurenitril zu über 95 % umgesetzt und mit Selektivitäten bis 92 % in 5-Oxohexansäure umgewandelt.

Eine besonders vorteilhafte Ausführungsform ist die folgende : Man verwendet Schwefelsäure als Mineralsäure und führt die nach der eben beschriebenen Extraktion erhaltene wäßrige Phase, bestehend aus Wasser, Schwefelsäure und deren Ammoniumsalzen in die Reaktion zurück, unter Ergänzung der fehlenden Mengen an Schwefelsäure und Wasser, dh. 1 Mol Schwefelsäure und 2 Mol Wasser pro Mol im vorhergehenden Durchgang umgesetztes Nitril. Bei mehrmaligem Wiederholen dieser Verfahrensweise reichern sich die Ammoniumsalze der Schwefelsäure in der wäßrigen Phase soweit an, daß schließlich eine heiß gesättigte Lösung entsteht, aus der die Salze beim Abkühlen auskristallisieren und in fester Form abgetrennt werden können.

Die letztgenannte Verfahrensweise besitzt den Vorteil, daß praktisch keine umweltbelastenden, schwefelsauren und Ammoniumsalze enthaltenden Abwässer anfallen, sondern die Ammoniumsalze kristallin isolierbar sind. Weitere Vorteile bestehen darin, daß die Selektivität der Bildung von 5-Oxohexansäure auf über 95 % gesteigert wird und die Verfahrensweise für die Übertragung auf einen kontinuierlichen Verfahrensablauf geeignet ist.

Die folgenden Beispiele sollen die Erfindung näher erläutern.

## Beispiele 1 bis 3

1 Mol 5-Oxohexansäurenitril (OHN) wird vorgelegt und unter Rühren tropfenweise mit einem Gemisch aus 1,5 Mol konzentrierter Schwefelsäure und 5 Mol Wasser versetzt, wobei die Zutropfzeit, die Gesamtreaktionszeit und die Reaktionstemperatur variiert werden (siehe Tabelle 1). Nach erfolgter Reaktion wird das heiße Reaktionsgemisch mit Chlorbenzol extrahiert, die organische Phase im Dünnschichtverdampfer von Extraktionsmittel befreit und anschließend alle organischen Bestandteile bei 130 °C und 1 mbar von anorganischen Anteilen abdestilliert. Die gaschromatografische Analyse ergab die in Tabelle 1 angegebenen Umsatz- und Selektivitätswerte.

Tabelle 1

| Beispiel | Zutropfzeit (min) | Gesamtreaktion Seit (min) | T (°C) | % Umsatz OHN | % Selektivität OHS |
|---|---|---|---|---|---|
| 1 | 30 | 60 | 120 | 90 | 90 |
| 2 | 45 | 90 | 115 | 96 | 89 |
| 3 | 90 | 180 | 80 | 61 | 92 |

## Beispiele 4 bis 6

Zu 2 Mol vorgelegtem 5-Oxohexansäurenitril (OHN) wird ein Gemisch aus 3 Mol Mineralsäure und 10 Mol Wasser zugetropft. Als Mineralsäure wird Schwefelsäure, Phosphorsäure bzw. Salzsäure verwendet. Die Reaktionszeit für jede Umsetzung beträgt 90 min, die Reaktionstemperatur 115 °C. Die Reaktionsgemische werden gemäß den Beispielen 1 bis 3 aufgearbeitet und analysiert. Die Ergebnisse der Bildung von 5-Oxohexansäure (OHS) sind in Tabelle 2 zusammengefaßt.

Tabelle 2

| Beispiel | Säure | % Umsatz OHN | % Selektivität OHS |
|---|---|---|---|
| 4 | Schwefelsäure | 95 | 90 |
| 5 | Salzsäure | 93 | 85 |
| 6 | Phosphorsäure | 45 | 78 |

## Beispiel 7

4 Mol 5-Oxohexansäurenitril werden vorgelegt und unter Rühren innerhalb von 45 min mit einem Gemisch aus 6 Mol konzentrierter Schwefelsäure und 40 Mol Wasser tropfenweise versetzt. Die unter Selbsterwärmung erzielte Temperatur von 110 °C wird weitere 45 min lang gehalten und danach das heiße Reaktionsgemisch zweimal mit je 1 l Chlorbenzol extrahiert. Aus den organischen Phasen wird anschließend das Chlorbenzol destillativ zurückgewonnen und die organischen Bestandteile durch Destillation im Dünnschichtverdampfer bei 130 °C und 1 mbar von anhaftenden anorganischen Anteilen befreit. Die wäßrige Phase wird mit 4 Mol konzentrierter Schwefelsäure versetzt und mit 4 Mol 5-Oxohexansäurenitril wieder zur Reaktion gebracht und anschließend genauso wie vorher aufgearbeitet. Die Reaktion, Aufarbeitung und Rückführung der wäßrigen, mit Schwefelsäure aufkonzentrierten Phase erfolgt noch zweimal, wobei zuvor durch Abkühlen der wäßrigen Phase auf etwa 20 °C zunehmende Mengen an Ammoniumsalzen der Schwefelsäure auskristallisieren und abgetrennt werden. Nach der vierten Umsetzung kristallisiert eine dem Umsatz an 5-Oxohexansäurenitril von ca. 98 % entsprechende Menge an Ammoniumsalzen aus. In den folgenden 26 Umsetzungen wurden daraufhin je 4 Mol des Nitrils unter Zusatz von je 4 Mol konzentrierter Schwefelsäure, je 8 Mol Wasser und der jeweiligen wäßrigen Phase zur Reaktion gebracht. Sowohl die Ammoniumsalzmengen als auch die von Chlorbenzol befreiten organischen Phasen der 26 Umsetzungen wurden gesammelt. Aus 11,5 kg 5-Oxohexansäurenitril waren 12,6 kg 5-Oxohexansäure und 12,5 kg Ammoniumsalze entstanden. 0,25 kg des Nitrils wurden zurückgewonnen, so daß der Nitril-Umsatz 98 %, die Selektivität zu 5-Oxohexansäure 95 % betrug.

## Ansprüche

1. Verfahren zur Herstellung von 5-Oxohexansäure, dadurch gekennzeichnet, daß 5-Oxohexansäurenitril mit 2 bis 30 Mol Wasser und 0,1 bis 5 Mol einer starken Mineralsäure je Mol 5-Oxohexansäurenitril bei Temperaturen von 20 bis 200 °C umgesetzt wird.

2. Verfahren nach Anspruch 1, dadurch gekennzeichnet, daß 2 bis 15 Mol Wasser je Mol 5-Oxohexansäurenitril verwendet werden.

3. Verfahren nach Anspruch 1, dadurch gekennzeichnet, daß 2 bis 5 Mol Wasser je Mol 5-Oxohexansäurenitril verwendet werden.

4. Verfahren nach einem der Ansprüche 1 bis 3, dadurch gekennzeichnet, daß 0,5 bis 2 Mol einer

**0 062 305**

starken Mineralsäure je Mol 5-Oxohexansäurenitril verwendet werden.

5. Verfahren nach einem der Ansprüche 1 bis 3, dadurch gekennzeichnet, daß 0,75 bis 1,5 Mol einer starken Mineralsäure je Mol 5-Oxohexansäurenitril verwendet werden.

6. Verfahren nach einem der Ansprüche 1 bis 5, dadurch gekennzeichnet, daß die Temperaturen 50 bis 150 °C betragen.

7. Verfahren nach einem der Ansprüche 1 bis 5, dadurch gekennzeichnet, daß die Temperaturen 80 bis 130 °C betragen.

8. Verfahren nach einem der Ansprüche 1 bis 7, dadurch gekennzeichnet, daß als starke Mineralsäure Schwefelsäure, Salzsäure oder Phosphorsäure verwendet wird.

9. Verfahren nach einem der Ansprüche 1 bis 7, dadurch gekennzeichnet, daß als starke Mineralsäure Schwefelsäure verwendet wird.

10. Verfahren nach Anspruch 9, dadurch gekennzeichnet, daß man aus dem Reaktionsgemisch durch Extraktion mit einem mit Wasser nicht mischbaren organischen Lösungsmittel die organischen Bestandteile abtrennt, aus dem Extrakt die 5-Oxohexansäure destillativ isoliert und die wäßrigmineralsäure Phase in die Reaktion zurückführt, nachdem man die gebildeten Ammoniumsalze der Schwefelsäure durch Kühlen in kristalliner Form abgetrennt hat.

11. Verfahren nach Anspruch 10, dadurch gekennzeichnet, daß man aus dem Extrakt nicht umgesetztes 5-Oxohexansäurenitril isoliert und erneut mit Wasser und Mineralsäure umsetzt.

**Claims**

1. A process for the preparation of 5-oxohexanoic acid, characterized by reacting 5-oxohexanenitrile with 2 to 30 moles of water and 0.1 to 5 moles of a strong mineral acid per mole of 5-oxohexanenitrile at temperatures from 20 to 200 °C.

2. The process as claimed in claim 1, wherein 2 to 15 moles of water are employed per mole of 5-oxohexanenitrile.

3. The process as claimed in claim 1, wherein 2 to 5 moles of water are employed per mole of 5-oxohexanenitrile.

4. The process as claimed in any of claims 1 to 3, wherein 0.5 to 2 moles of a strong mineral acid are employed per mole of 5-oxohexanenitrile.

5. The process as claimed in any of claims 1 to 3, wherein 0.75 to 1.5 mole of a strong mineral acid are employed per mole of 5-oxohexanenitrile.

6. The process as claimed in any of claims 1 to 5, wherein the temperatures are 50 to 150 °C.

7. The process as claimed in any of claims 1 to 5, wherein the temperatures are 80 to 130 °C.

8. The process as claimed in any of claims 1 to 7, wherein sulfuric acid, hydrochloric acid or phosphoric acid is employed as the strong mineral acid.

9. The process as claimed in any of claims 1 to 7, wherein sulfuric acid is employed as the strong mineral acid.

10. The process as claimed in claim 9, which comprises separating off the organic constituents from the reaction mixture by extraction with a water-immiscible organic solvent, isolating 5-oxohexanoic acid from the extract by distillation and returning the aqueous mineral acid phase to the reaction, after the resulting ammonium salts of sulfuric acid have been separated off in crystalline form by cooling.

11. The process as claimed in claim 10, wherein unreacted 5-oxohexanenitrile is isolated from the extract and reacted again with water and mineral acid.

**Revendications**

1. Procédé de préparation de l'acide oxo-5 hexanoïque, procédé caractérisé en ce qu'on fait réagir l'oxo-5 hexane-nitrile avec de 2 à 30 moles d'eau et de 0,1 à 5 moles d'un acide minéral fort par mole d'oxo-5 hexane-nitrile, à des températures comprises entre 20 et 200 °C.

2. Procédé selon la revendication 1, caractérisé en ce qu'on utilise de 2 à 15 moles d'eau par mole d'oxo-5 hexane-nitrile.

3. Procédé selon la revendication 1, caractérisé en ce qu'on utilise de 2 à 5 moles d'eau par mole d'oxo-5 hexane-nitrile.

4. Procédé selon l'une quelconque des revendications 1 à 3, caractérisé en ce qu'on utilise de 0,5 à 2 moles d'un acide minéral fort par mole d'oxo-5 hexane-nitrile.

5. Procédé selon l'une quelconque des revendications 1 à 3, caractérisé en ce qu'on utilise de 0,75 à 1,5 mole d'un acide minéral fort par mole d'oxo-5 hexane-nitrile.

6. Procédé selon l'une quelconque des revendications 1 à 5, caractérisé en ce que les températures sont comprises entre 50 et 150 °C.

7. Procédé selon l'une quelconque des revendications 1 à 5, caractérisé en ce que les températures sont comprises entre 80 et 130 °C.

8. Procédé selon l'une quelconque des revendications 1 à 7, caractérisé en ce qu'on utilise, comme

4

**0 062 305**

acide minéral fort, l'acide sulfurique, l'acide chlorhydrique ou l'acide phosphorique.

9. Procédé selon l'une quelconque des revendications 1 à 7, caractérisé en ce qu'on utilise, comme acide minéral fort, l'acide sulfurique.

10. Procédé selon la revendication 9, caractérisé en ce qu'on sépare les constituants organiques du mélange réactionnel par extraction avec un solvant organique non miscible à l'eau, on isole de l'extrait, par distillation, l'acide oxo-5 hexanoïque et, après avoir séparé à l'état cristallisé, par refroidissement, les sels d'ammonium de l'acide sulfurique qui se sont formés, on renvoie à la réaction la phase aqueuse qui est acide du fait de la présence d'un acide minéral.

11. Procédé selon la revendication 10, caractérisé en ce qu'on isole de l'extrait l'oxo-5 hexane-nitrile qui n'a pas réagi et on le fait à nouveau réagir avec l'eau et l'acide minéral.